# DEMANDE DE BREVET EUROPEEN

(11) **EP 1 108 415 A2**
(43) Date de publication de la demande: **20.06.2001**
(21) Numéro de dépôt: 00403236.3
(22) Date de dépôt: 20.11.2000
(51) Int. Cl.: A61K 7/032, A61K 7/48

(54) **Composition cosmétique comprenant une cire**

(30) Priorité: 30.11.1999 FR 9915098
(71) Demandeur: L'OREAL, 75008 Paris (FR)
(72) Inventeur: Auguste, Frédéric, 94550 Chevilly-Larue (FR)
(74) Mandataire: Kromer, Christophe

(57) **Abrégé**

L'invention concerne une composition à application topique comprenant, dans un milieu physiologiquement acceptable, au moins une phase grasse liquide, contenant au moins une huile et une cire ayant un point de fusion inférieur à 60 °C et une dureté, à 20 °C, supérieure ou égal à 9,5 MPa.

L'invention a aussi pour objet un procédé cosmétique de maquillage ou de tria-teent non thérapeutique des matières kératiniques comprenant l'application sur les matières kératinques d'une telle composition.

## Description

La présente invention a pour objet une composition à application topique contenant une phase grasse liquide et une cire, destinée en particulier au domaine cosmétique. Plus précisément, l'invention se rapporte à une composition pour le maquillage ou le soin des matières kératiniques telles que la peau, y compris les lèvres, les fibres kératiniques comme les cils, les sourcils, les cheveux, et les ongles, notamment d'êtres humains. L'invention a également pour objet un procédé de maquillage ou de soin des matières kératiniques, ainsi que l'utilisation de la composition pour recourber les fibres kératiniques.

Cette composition peut se présenter notamment sous forme de composition de revêtement des fibres kératiniques, d'eye-liner, de produit de maquillage pour le corps, de produit anti-cernes, de fard à paupières ou à joues, de fond de teint, de rouge à lèvres, de vernis à ongles, de composition de protection solaire, de coloration de la peau, de produit de coiffage.

La composition de revêtement des fibres kératiniques peut être une composition de maquillage, une base de maquillage, une composition à appliquer sur un maquillage, dite encore top-coat, ou bien encore une composition de traitement cosmétique des fibres kératiniques. Plus spécialement, l'invention porte sur un mascara.

Les compositions de maquillage des fibres kératiniques comme les mascaras contiennent généralement, de façon connue, des cires qui forment un dépôt destiné à gainer les fibres kératiniques après l'application de la composition. Pour obtenir un maquillage résistant à l'eau, dit aussi waterproof, il est connu d'incorporer les cires dans une phase grasse liquide, telle que l'isododécane.

Toutefois, lorsque des cires dures comme la cire de carnauba ou la cire de candelilla sont incorporées dans l'isododécane à une teneur d'au moins 10 % en poids par rapport au poids total du mélange de cire et de phase grasse liquide, on constate un épaississement important du mélange qui présente alors une consistance solide. Cet épaississement ne facilite pas la mise en oeuvre de ces cires en quantité importante dans la composition. De plus, la composition épaisse ne s'applique pas aisément sur les matières kératiniques, notamment les cils, et a tendance à former des paquets : le maquillage n'est pas homogène, ce qui nuit à l'effet recherché et laisse une sensation de gêne et d'inconfort à l'utilisatrice.

Le but de la présente invention est donc d'obtenir une composition à application topique pouvant comporter une quantité importante de cire dans une phase grasse liquide tout en évitant un épaississement trop important de la composition.

Les inventeurs ont découvert qu'une telle composition pouvait être obtenue en utilisant des cires à bas point de fusion et ayant une dureté élevée, mélangées avec une phase grasse liquide. La composition peut comprendre jusqu'à 30 % en poids de cire sans présenter une consistance solide. La composition s'applique facilement sur les matières kératiques, conduisant à un maquillage homogène. De plus, lorsque la composition est appliquée sur les fibres kératiniques telles que les cils, elle permet d'obtenir un recourbement important des fibres kératiniques.

De façon plus précise, la présente invention a pour objet une composition à application topique comprenant, dans un milieu physiologiquement acceptable, au moins une phase grasse liquide contenant au moins une huile et au moins une cire, caractérisée par le fait que la cire a un point de fusion inférieur à 60 °C et une dureté, à 20 °C, supérieure ou égale à 9,5 MPa.

L'invention a également pour objet un procédé de maquillage ou de traitement non thérapeutique des matières kératiniques, comprenant l'application sur les matières kératiniques de la composition décrite précédemment.

L'invention a aussi pour objet l'utilisation d'une composition telle que définie précédemment pour recourber les fibres kératiniques, notamment les cils.

Par physiologiquement acceptable, il faut comprendre un milieu compatible avec la peau et/ou les fibres kératiniques, comme un milieu cosmétique.

Avantageusement, la cire peut avoir un point de fusion allant de 30 °C à 59 °C, de préférence allant de 35 °C à 59 °C, et mieux allant de 40 °C à 50 °C.

De préférence, la cire peut avoir une dureté allant de 9,5 MPa à 20 MPa, et mieux allant de 9,5 MPa à 15 MPa. Avantageusement, la cire peut avoir une durété supérieure à 10 MPa, notamment allant de 10 à 20 MPa, et mieux allant de 10 à 12 MPa.

Selon la présente demande, la dureté de la cire est déterminée par la mesure de la force en compression mesurée à 20 °C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

Comme cire répondant aux critères définis précédemment, on peut utiliser les composés de formule (I) suivante : 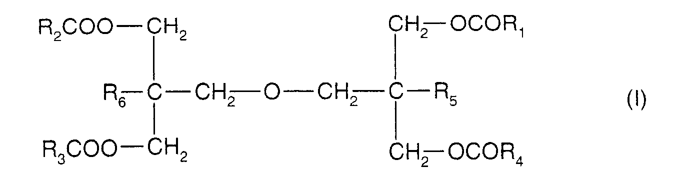 dans laquelle :
- R₁, R₂, R₃ et R₄ désignent, indépendamment l'un de l'autre, un radical alkyl linéaire ayant de 13 à 19 atomes de carbone, et de préférence de 15 à 17 atomes de carbone,
- R₅ et R₆ désignent, indépendamment l'un de l'autre, un radical alkyle choisi parmi les radicaux méthyle, éthyle et propyle.

De préférence, R₁, R₂, R₃ et R₄ désignent un radical alkyle identique et notamment un radical alkyl ayant 17 atomes de carbones.

R₅ et R₆ désignent avantageusemnent le radical méthyle.

Les composés de formule (I) peuvent être obtenus par réaction d'un polyol de formule (II) 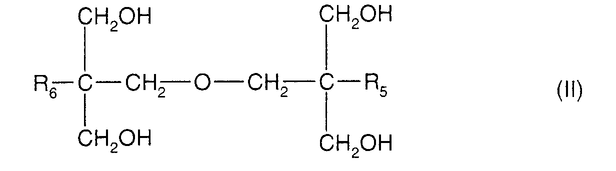 avec un acide carboxylique R-COOH,
R₅ et R₆ ayant la même signification que celle indiquée précédemment et R désignant un radical R₁, R₂, R₃ ou R₄ tels que définis précédemment.

Un composé de formule (I) particulièrement préféré est le tétrastéarate de di(triméthylol-1,1,1 propane) correspondant aux significations R₁ = R₂ = R₃ = R₄ = radical alkyl en C17, R₅ = R₆ = radical éthyle. Ce composé est vendu sous la dénomination HEST 2T- 4S par la société HETERENE.

On peut également utiliser la cire obtenue par hydrogénation d'huile d'olive estérifiée avec l'alcool stéarylique qui est vendue sous la dénomination PHYTOWAX Olive 18 L 57 par la société SOPHIM.

La cire peut être présente dans la composition selon l'invention en une teneur allant de 0,5 % à 60 % en poids, par rapport au poids total de la composition, de préférence de 1 % à 40 % en poids, mieux de 2 % à 30 % en poids, et encore mieux de 2 % à 20 % en poids.

L'huile présente dans la phase grasse de la composition selon l'invention peut être choisie parmi les huiles volatiles et les huiles non volatiles. La phase grasse de la composition peut comprendre de préférence une huile volatile.

On entend par "huile volatile" une huile susceptible de s'évaporer à température ambiante d'un support sur lequel elle a été appliquée, autrement dit une huile ayant une tension de vapeur mesurable à température ambiante.

On peut notamment utiliser une ou plusieurs huiles volatiles à température ambiante et pression atmosphérique ayant par exemple une tension de vapeur, à pression et température ambiante > 0 mm de Hg (0 Pa) et en particulier allant de 10⁻³ à 300 mm de Hg (0,13 Pa à 40.000 Pa), à condition que la température d'ébullition soit supérieure à 30°C. Ces huiles volatiles sont favorables à l'obtention d'un film à propriétés "sans transfert" total et de bonne tenue. Ces huiles volatiles facilitent, en outre, l'application de la composition sur la peau, les muqueuses, les phanères. Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles préférées convenant pour la composition selon l'invention sont en particulier les huiles hydrocarbonées ayant de 8 à 16 atomes de carbones, et notamment les isoalcanes en C₈-C₁₆ (ou isoparaffines) et les esters ramifiés en C₈-C₁₆ comme l'isododécane (encore appelé 2,2,4,4,6-pentaméthylheptane), l'isodécane, l'iso-hexadécane, le néo pentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées.

Comme huiles volatiles, on peut aussi utiliser les silicones volatiles, comme par exemple les huiles de silicones cycliques et volatiles, notamment celles ayant une viscosité ≤ 8 centistokes (8, 10⁻⁶ m²/s), telles que l'octaméthylcyclotétrasiloxane, le décaméthylcyclopentasiloxane, le dodécaméthylcyclohexasiloxane, les silicones linéaires volatiles telles que l'octaméthyltrisiloxane, l'heptaméthylhexyltrisiloxane, l'heptaméthyloctyltrisiloxane, le décaméthyltétrasiloxane, ou bien encore les huiles volatiles fluorées telles que le nonafluorométhoxybutane ou le perfluorométhylcyclopentane.

L'huile volatile peut être présente dans la composition selon l'invention en une teneur allant de 1 % à 99,5 % en poids, par rapport au poids total de la composition, de préférence de 5 % à 98 % en poids, mieux de 30 % à 70 % en poids et encore mieux de 40 % à 60 % en poids.

La composition selon l'invention peut également contenir des huiles non volatiles, et notamment des huiles hydrocarbonées et/ou siliconées et/ou fluorées non volatiles.
Comme huile hydrocarbonée non volatile, on peut notament citer :
- les huiles hydrocarbonées d'origine animale telle que le perhydrosqualène;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité;
- les hydrocarbures linéaires ou ramifiés, d'origine minérale ou synthétique tels que la vaseline, les polydécènes, le polyisobutène hydrogéné tel que le parléam;
- les esters et les éthers de synthèse comme les huiles de formule R₁₀COOR₁₁ dans laquelle R₁₀ représente le reste d'un acide gras supérieur comportant de 6 à 29 atomes de carbone et R₁₁ représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyldodécyle ; les esters de polyols comme le dioctanoate de propylène glycol, le diheptanoate de néopentylglycol, le diisononanoate de diéthylène glycol et les esters du pentaérythritol;
- les esters à chaîne courte ayant de 3 à 8 atomes de carbone au total tels que l'acététe d'éthyle, l'acétate de méthyle, l'acétate de propyle, l'acétate de n-butyle, l'acétate d'isopentyle ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide myristique, l'acide palmitique, l'acide stéarique, l'acide béhénique, l'acide oléique, l'acide linoléique, l'acide linolénique ou l'acide isostéarique ;
et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles non volatiles peuvent être présentes dans la composition selon l'invention en une teneur allant de 0 % à 20 % en poids (notamment 0,1 à 20 %), par rapport au poids total de la composition, de préférence de 0 % à 2 % en poids, et mieux de 0,1 % à 2 % en poids.

La composition selon l'invention peut comprendre, en outre, au moins une cire additionnelle différente de la cire décrite précédemment. La cire additionnelle peut être choisie parmi les cires ayant un point de fusion supérieur ou égal à 60 °C et les cires ayant un point de fusion inférieur à 60 °C et une dureté, à 20 °C, inférieure à 9,5 MPa. De préférence, la cire additionnelle a un point de fusion supérieur ou égal à 40 °C, et mieux allant de 60 °C à 110 °C.

La cire additionnelle peut être choisie parmi les cires d'origine animale, les cires d'origine végétale ou les cires d'origine synthétique.

Parmi les cires d'origine animale, on peut citer les cires d'abeille, les cires de lanoline et les cires d'insectes de Chine.
Parmi les cires d'origine végétale, on peut citer les cires de riz, les cires de Carnauba, de Candelilla, d'Ouricurry, les cires de fibres de liège, les cires de canne à sucre, les cires du Japon, la cire de Sumac, la cire de coton.
Parmi les cires d'origine minérale, on peut citer les paraffines, les cires microcristallines, les cires de Montan et les ozokérites.
Parmi les cires d'origine synthétique, on peut utiliser notamment les cires de polyoléfine et notamment les cires de polyéthylène, les cires obtenues par la synthèse de Fischer et Tropsch, les copolymères cireux ainsi que leurs esters, les cires de silicone.
Il est également possible d'utiliser des huiles d'origine animale ou végétale hydrogénées qui répondent toujours aux deux caractéristiques physiques mentionnées précédemment.
Parmi ces huiles, on peut citer les huiles hydrogénées qui sont obtenues par hydrogénation catalytique de corps gras composés de chaîne grasse linéaire ou non en C₈-C₃₂, l'huile de tournesol hydrogénée, l'huile de ricin hydrogénée, l'huile de coprah hydrogénée, l'huile de jojoba hydrogénée, la lanoline hydrogénée et les huiles de palme hydrogénées.
Les cires additionnelles utilisables dans la composition selon l'invention sont de préférence solides et rigides à température inférieure à 50 °C.

La composition selon l'invention peut comprendre de 0 % à 30 % (notamment de 0,1 % à 30 %) en poids de cire additionnelle, par rapport au poids total de la composition, de préférence de 0,5 % à 25 % en poids.

La composition selon l'invention peut contenir en outre, dans la phase grasse liquide, un polymère filmogène. Ce polymère filmogène peut notamment être soluble, ou dit encore liposoluble, dans la phase grasse liquide, ou bien encore être dispersé sous forme de particules dans la phase grasse liquide. Ce polymère filmogène confère notamment une bonne tenue de la composition après application sur les matières kératiniques.

On entend par "polymère filmogène" un polymère capable de former seul un film isolable.

A titre d'exemples de polymères filmogènes liposolubles, on peut citer les polymères correspondant à la formule (III) suivante : 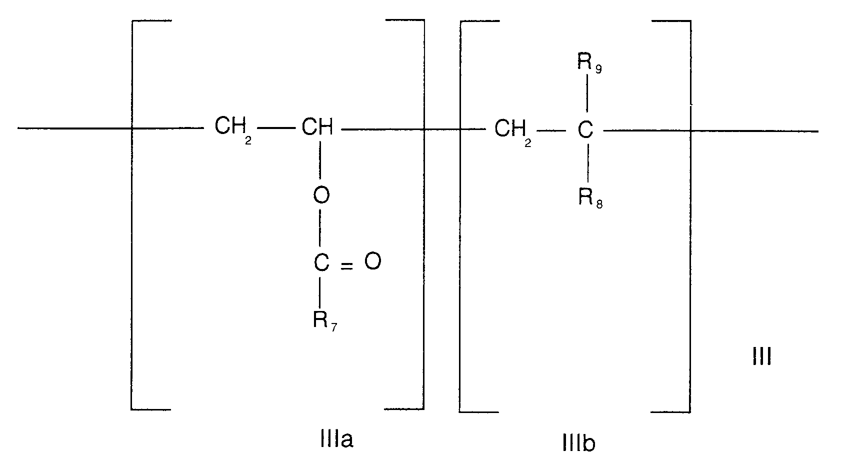 dans laquelle :
- R₇ représente une chaîne hydrocarbonée saturée, linéaire ou ramifiée, ayant de 1 à 19 atomes de carbone ;
- R₈ représente un radical pris dans le groupe constitué par :
   a) -O-CO-R₁₀, R₁₀ ayant la même signification que R₇ mais étant différent de R₇ dans un même copolymère,
   b) -CH₂-R₁₁, R₁₁ représentant une chaîne hydrocarbonée saturée, linéaire ou ramifiée, ayant de 5 à 25 atomes de carbone,
   c) -O-R₁₂, R₁₂ représentant une chaîne hydrocarbonée saturée, ayant de 2 à 18 atomes de carbone, et
   d) -CH₂-O-CO-R₁₃, R₁₃ représentant une chaîne hydrocarbonée saturée, linéaire ou ramifiée, ayant de 1 à 19 atomes de carbone,
- R₉ représente un atome d'hydrogène quand R₈ représente les radicaux a), b) ou c) ou R₉ représente un radical méthyle quand R₈ représente le radical d), ledit copolymère devant être constitué d'au moins 15 % en poids d'au moins un monomère dérivé d'un motif (Illa) ou d'un motif (IIIb) dans lesquels les chaînes hydrocarbonées saturées, linéaires ou ramifiées, ont au moins 7 atomes de carbone.

Les copolymères de formule (III) résultent de la copolymérisation d'au moins un ester vinylique (correspondant au motif Illa)) et d'au moins un autre monomère (correspondant au motif IIIb)) qui peut être une α-oléfine, un alkylvinyléther ou un ester allylique ou méthallylique.

Lorsque dans le motif (IIIb)) R₈ est choisi parmi les radicaux -CH₂-R₁₁, -O-R₁₂ ou -CH₂-O-CO-R₁₃ tels que définis précédemment, le copolymère de formule (III) peut être constitué de 50 à 95 % en moles d'au moins un motif (Illa)) et de 5 à 50 % en moles d'au moins un motif (Illb).

Les copolymères de formule (III) peuvent également résulter de la copolymérisation d'au moins un ester vinylique et d'au moins un autre ester vinylique différent du premier. Dans ce cas, ces copolymères peuvent être constitués de 10 à 90 % en moles d'au moins un motif (Illa) et de 10 à 90% en moles d'au moins un motif (Illb) dans lequel R₈ représente le radical -O-CO-R₁₀.

Parmi les esters vinyliques conduisant au motif de formule (Illa), ou au motif de formule (Illb) dans lequel R₈ = -O-CO-R₁₀, on peut citer l'acétate de vinyle, le propionate de vinyle, le butanoate de vinyle, l'octanoate de vinyle, le décanoate de vinyle, le laurate de vinyle, le stéarate de vinyle, l'isostéarate de vinyle, le diméthyl-2, 2 octanoate de vinyle, et le diméthylpropionate de vinyle.

Parmi les α-oléfines conduisant au motif de formule (IIIb) dans lequel R₈ = -CH₂-R₁₁, on peut citer l'octène-1, le dodécène-1, l'octadécène-1, l'eicosène-1, et les mélanges d' α-oléfines ayant de 22 à 28 atomes de carbone.

Parmi les alkylvinyléthers conduisant au motif de formule (Illb) dans lequel R₈ = -O-R₁₂, on peut citer l'éthylvinyléther, le n-butylvinyléther, l'isobutylvinyléther, le décylvinyléther, le dodécylvinyléther, le cétylvinyléther et l'octadécylvinyléther.

Parmi les esters allyliques ou méthallyliques conduisant au motif de formule (IIIb) dans lequel R₈ = -CH₂-O-CO-R₁₃, on peut citer les acétates, les propionates, les diméthylpropionates, les butyrates, les hexanoates, les octanoates, les décanoates, les laurates, les diméthyl-2, 2 pentanoates, les stéarates et les eicosanoates d'allyle et de méthallyle.

Les copolymères de formule (III) peuvent également être réticulés à l'aide de certains types de réticulants qui ont pour but d'augmenter sensiblement leur poids moléculaire.

Cette réticulation est effectuée lors de la copolymérisation et les réticulants peuvent être soit du type vinylique, soit du type allylique ou méthallylique. Parmi ceux-ci, on peut citer en particulier le tétraallyloxyéthane, le divinylbenzène, l'octanedioate de divinyle, le dodécanedioate de divinyle, et l'octadécanedioate de divinyle.

Parmi les différents copolymères de formule (III) utilisables dans la composition selon l'invention, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2262303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, différents de la cire de polyoléfine définie en a), comme le polybutène, les alkylcelluloses avec un radical alkyle linéaire ou ramifié, saturé ou non en C₁ à C₈ comme l'éthylcellulose et la propylcellulose, les copolymères de la vinylpyrrolidone (VP) et notamment les copolymères de la vinylpyrrolidone et d'alcène en C₂ à C₄₀ et mieux en C₃ à C₂₀. A titre d'exemples de copolymères de VP utilisable dans l'invention, on peut citer le copolymère de VP/acétate de vinyle, VP/méthacrylate d'éthyle, la polyvinylpyrrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène liposoluble peut être présent dans la composition en une teneur allant de 0,1 % à 20 % en poids, par rapport au poids total de la composition, et de préférence de 0,5 % à 15 % en poids.

La composition selon l'invention peut comprendre, en outre, une dispersion stable de particules d'un système polymérique filmifiable ou non filmifiable, les particules étant stabilisées en surface dans la phase grasse liquide. La composition peut d'ailleurs comprendre un mélange de dispersion stable d'un premier système polymérique filmifiable et d'un second système polymérique non filmifiable.

Les particules du système polymérique ont de préférence une taille allant de 5 nm à 600 nm, et de préférence de 50 nm à 250 nm. Ces particules sont généralement de forme sensiblement sphérique.

On entend par "système polymérique filmifiable" un système polymérique apte à former un film isolable à température ambiante (25 °C). Le premier système polymérique filmogène a de préférence une température de transition vitreuse (Tg 1) basse, inférieure ou égale à la température de la peau, et en particulier inférieure ou égale à environ 40 °C. Avantageusement, la Tg 1 va d'environ -100 °C à environ 40 °C, et mieux va de - 10 °C à 30 °C.

On entend par "système polymérique non filmifiable", un système polymérique qui ne permet pas de former un film isolable à température ambiante (25 °C). Le deuxième système polymérique non filmifiable permet toutefois, en association avec la phase grasse liquide, de former un dépôt continu et homogène sur les matières kératiniques. Le deuxième système polymérique non filmifiable a de préférence une température de transition vitreuse (Tg 2) supérieure à environ 40 °C, et en particulier inférieure ou égale à 300 °C. Avantageusement, la Tg 2 va environ de 45 °C à 150 °C.

La mesure de Tg (température de transition vitreuse) est effectuée par DSC (Differential Scanning Calorimetry) selon la norme ASTM D3418-97.

Selon un premier mode de réalisation de la composition selon l'invention, le système polymérique peut consister essentiellement en un polymère ayant les caractéristiques du système polymérique décrites précédemment.

Lorsque le polymère ne permet pas d'obtenir seul les caractéristiques du système polymérique mentionnées précédemment, il est possible d'ajouter un composé dont la fonction est de modifier les propriétés de chaque polymère pour obtenir le système polymérique souhaité. Aussi, selon un second mode de réalisation de la composition selon l'invention, on peut ajouter au polymère au moins un agent plastifiant permettant d'obtenir un système polymérique ayant les caractéristiques telles que décrites précédemment. Dans ce cas, le système polymérique comprend un mélange de polymère et d'au moins un agent plastifiant.

Le ou les plastifiants peuvent être choisis parmi les plastifiants usuellement utilisés dans le domaine d'application et notamment parmi les composés susceptibles d'être des solvants du polymère.

Le polymère en dispersion dans la phase grasse liquide peut être de toute nature. Parmi les polymères utilisables, on peut citer les polymères radicalaires, les polycondensats, et les polymères d'origine naturelle et leurs mélanges. Les polymères peuvent être choisis par l'homme du métier en fonction de leurs propriétés.

Les polymères en dispersion utilisables dans la composition de l'invention ont de préférence un poids moléculaire moyen en poids allant de l'ordre de 2000 à 10 000 000.

Par polymère radicalaire, on entend un polymère obtenu par polymérisation d'un ou plusieurs monomères à insaturation notamment éthylénique, chaque monomère étant susceptible de s'homopolymériser (à l'inverse des polycondensats). Les polymères radicalaires peuvent être notamment des polymères ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique et leurs associations. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆ .
Parmi les (méth)acrylates d'alkyle, on peut citer le (méth)acrylate de méthyle, le (méth)acrylate d'éthyle, le (méth)acrylate de butyle, le (méth)acrylate d'isobutyle, le (méth)acrylate d'éthyl-2 hexyle, le (méth)acrylate de lauryle.
Parmi les (méth)acrylates d'hydroxyalkyle, on peut citer le (méth)acrylate d'hydroxyéthyle, le (méth)acrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Comme polymère radicalaire, on utilise de préférence les copolymères d'acide (méth)acrylique et de (méth)acrylate d'alkyle, notamment d'alkyle en C₁-C₄. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth)acrylamides, en particulier d'alkyl en C₂-C₁₂ tels que le N-éthyl acrylamide, le N-t-butyl acrylamide, le N-octyl acrylamide ; les N,N- dialkyl (C₁-C₄) (méth)acrylamides.

Les polymères vinyliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyl, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemples d'esters vinyliques, on peut citer l'acétate de vinyle, le propionate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques, on peut encore citer :
- la N-vinylpyrrolidone; la vinylcaprolactame; les vinyl N-alkyl(C₁-C₆) pyrroles; les vinyl-oxazoles; les vinyl-thiazoles; les vinylpyrimidines; les vinylimidazoles;
- les oléfines tels que l'éthylène, le propylène, le butylène, l'isoprène, le butadiène.

Le polymère vinylique peut être réticulé à l'aide de monomère difonctionnel, notamment comprenant au moins deux insaturations éthyléniques, tel que le diméthacrylate d'éthylène glycol, le phtalate de diallyle.

Avec de telles dispersions de particules de polymère, il est possible de calibrer à volonté la taille des particules de polymère, et de moduler leur "polydispersité" en taille lors de la synthèse. Il est ainsi possible d'obtenir des particules de très petite taille, qui sont invisibles à l'oeil nu lorsqu'elles sont dans la composition et lorsqu'elles sont appliquées sur la peau ou les lèvres. Ceci ne serait pas possible avec des pigments sous forme particulaire, leur constitution ne permettant pas de moduler la taille moyenne des particules.

De façon non limitative, le polymère en dispersion peut être choisi parmi, les polymères ou copolymères suivants : polyuréthannes, polyuréthannes-acryliques, polyurées, polyurée-polyuréthannes, polyester-polyuréthannes, polyéther-polyuréthannes, polyesters, polyesters amides, polyesters à chaîne grasse, alkydes ; polymères siliconés, polymères fluorés et leurs mélanges.

Le polymère dispersé peut être utilisé en une quantité efficace pour obtenir un film continu et homogène, déposé sur les matières kératiniques et plus spécialement les fibres kératiniques, brillant et/ou non collant et/ou ayant des propriétés de non transfert.

En pratique, le polymère dispersé dans la phase grasse liquide peut être présent en une teneur totale allant de 2 % à 60 % en poids, par rapport au poids total de la composition, de préférence de 4 % à 40 % en poids, et mieux de 5 % à 30 % en poids.

Les particules de polymère sont stabilisées en surface, au fur et à mesure de la polymérisation, grâce à un stabilisant qui peut être notamment un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors de la polymérisation.

Le stabilisant est de préférence également présent dans le mélange avant polymérisation. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

Le stabilisant peut aussi être choisi parmi les copolymères blocs greffés ou séquencés comprenant au moins un bloc de type polyorganosiloxane et au moins un bloc d'un polyéther. Le bloc polyorganopolysiloxane peut être notamment un polydiméthylsiloxane ou bien encore un poly alkyl(C₂-C₁₈) méthyl siloxane ; le bloc polyéther peut être un poly alkylène en C₂-C₁₈, en particulier polyoxyéthylène et/ou polyoxypropylène. En particulier, on peut utiliser les diméthicones copolyols ou encore les alkyl (C₂-C₁₈) méthicones copolyols. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200" par la société DOW CORNING.

Comme copolymères blocs greffés ou séquencés, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly(méthylacrylate de méthyle)/polyisobutylène ou les copolymères greffés à squelette poly(méthacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.

On peut également employer des copolymères de (méth)acrylates d'alkyl en C₁-C₄, et de (méth)acrylates d'alkyl en C₈-C₃₀. On peut en particulier citer le copolymère méthacrylate de stéaryle/méthacrylate de méthyle.

Lorsqu'on utilise un polymère statistique en tant que stabilisant, on le choisit de manière à ce qu'il possède une quantité suffisante de groupements le rendant soluble dans le solvant de synthèse envisagé.

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxy (C₂-C₁₈)alkylène et notamment polyoxy-propylénés et/ou polyoxyéthylénés.

Lorsque la phase grasse liquide ne comprend pas d'huile de silicone, l'agent stabilisant est de préférence choisi dans le groupe constitué par :
- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyl en C₁-C₄, et d'acrylates ou de méthacrylates d'alkyl en C₈-C₃₀,
- (c) les copolymères blocs greffés ou séquencés comprenant au moins un bloc résultant de la polymérisation d'au moins un monomère éthylénique, à liaisons éthyléniques conjuguées,
et au moins un bloc d'un polymère vinylique ou acrylique ou d'un polyéther ou d'un polyester, ou leurs mélanges.

On peut bien entendu utiliser des associations des stabilisants décrits précédemment.

De préférence, on utilise des polymères dibloc comme agent stabilisant.

Suivant l'application, on pourra choisir d'utiliser des dispersions de polymères filmifiables ou non filmifiables, dans des huiles volatiles ou non volatiles.

Des dispersions de polymère dans une phase grasse liquide sont décrites dans les documents EP-A-749746, EP-A-923928, EP-A-930060 dont le contenu est incorporé à titre de référence dans la présente demande.

La composition selon l'invention peut comprendre, en outre, des gommes de silicones. Les gommes de silicone peuvent être des polysiloxanes de masse moléculaire élevée, de l'ordre de 200 000 à 1 000 000 et ayant une viscosité supérieure à 500 000 mPa.s. Elles peuvent être utilisées seules ou en mélange avec un solvant tel qu'une huile polydiméthylsiloxane ou polyphénylsiloxane.
Les gommes peuvent être présentes dans la composition en une teneur allant de 0 % à 2 % en poids, par rapport au poids total de la composition, et de préférence de 0,1 % à 1 % en poids.

La composition selon l'invention peut comprendre, en outre, un agent épaississant de la phase grasse liquide. L'agent épaississant peut être choisi parmi les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX.

L'agent épaississant peut être présent en une teneur allant de 0,5 % à 10 % en poids, par rapport au poids total de la composition, et mieux de 1 % à 6 % en poids.

La composition peut également comprendre au moins une matière colorante comme les composés pulvérulents et/ou les colorants liposolubles, par exemple à raison de 0,01 à 30 % du poids total de la composition. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert d'oxyde de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques.

La composition peut comprendre, en outre, tout additif habituellement utilisés dans de telles compositions, tels que les conservateurs, les parfums, les filtres solaires, les agents anti-radicaux libres, les agents hydratants, les vitamines, les protéines, les céramides, les plastifiants.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention est avantageusement anhydre ; elle peut aussi contenir jusqu'à 10 % en poids d'eau, notamment de 0,1 % à 10 % en poids, par rapport au poids total de la composition, formant une phase aqueuse. La phase aqueuse éventuellement présente dans la composition peut contenir un additif cosmétiquement ou dermatologiquement acceptable, ou bien encore polymère filmogène additionnel sous forme de particules dispersées dans la phase aqueuse.

La composition selon l'invention peut se présenter sous la forme fluide, gélifiée, semi-solide, pâte souple, voire solide telle que stick ou bâton. Ces formes galéniques sont préparées selon les méthodes usuelles des domaines considérés.

La composition selon l'invention peut être utilisée pour le maquillage ou le traitement cosmétique des matières kératiniques. La composition de maquillage peut être un eye-liner, un mascara, un fond de teint, un fard à paupières ou à joue, un rouge à lèvres, un produit anti-cernes, ou encore un produit de maquillage du corps du type tatouage provisoire ou semi-permanent. La composition de traitement cosmétique peut être une composition de soin pour le visage, le cou, les mains ou le corps ; elle peut constituer aussi une composition solaire ou autobronzante.

L'invention est illustrée plus en détail dans les exemples suivants.

### Exemple 1 : étude comparative

On a étudié la consistance de 4 cires (dont 2 selon l'invention) mélangées à différentes teneurs dans de l'isododécane.
- Cire 1 :: tétrastéarate de di-(triméthylol-1,1,1 propane) vendu sous la dénomination HEST 2T-4S par la société HETERENE
- Cire 2 :: cire vendue sous la dénomination PHYTOWAX Olive 18 L 57 par la société SOPHIM
- Cire 3 :: cire de carnauba
- Cire 4 :: cire de candelilla

Ces cires testées ont un point de fusion et une dureté suivantes :

| **Cire** | **Cire 1 (I)** | **Cire 2 (I)** | **Cire 3 (HI)** | **Cire 4 (HI)** |
|---|---|---|---|---|
| **Point de fusion (°C)** | 46 | 58 | 82 | 64 |
| **Dureté (MPa)** | 10,3 | 10 | 7 | 9,6 |
| I : invention ; HI : hors invention | | | | |

La dureté de chaque cire est mesurée à l'aide du texturomètre TA-TX2i de la société RHEO.

On a observé les consistances suivantes (les teneurs sont exprimées en pourcentage en poids, par rapport au poids total du mélange cire/isododécane) :

| **Cire** | **Cire 1 (I)** | **Cire 2 (I)** | **Cire 3 (HI)** | **Cire 4 (HI)** |
|---|---|---|---|---|
| **10 %** | liquide | liquide | solide | solide |
| **20 %** | gel épais | liquide | solide | solide |
| **30 %** | pâteux | pâteux | solide | solide |
| **50 %** | solide | solide | solide | solide |

On constate que les cires 1 et 2 selon l'invention peuvent être mélangées à l'isododécane jusqu'à une teneur d'au moins 30 % en poids sans que le mélange soit solide tandis que les cires 3 et 4 de l'art antérieur forment un mélange solide dès la teneur à 10 % en poids.

### Exemple 2 :

On a préparé un mascara ayant la composition suivante :
- Tétrastéarate de di-(triméthylol-1,1,1 propane) (HEST 2T-4S der la société HETERENE) 20 g
- Copolymère acétate de vinyle/stéarate d'allyle (65/35) (Mexomère PQ de CHIMEX) 7 g
- Quaternium-18 bentonite 5 g
- Propylène carbonate 1,6 g
- Oxyde de fer noir 5 g
- Isododécane qsp 100 g

La composition s'applique facilement sur les cils et confère à ceux-ci un très bon recourbement.

### Exemple 3 :

On a préparé un mascara ayant la composition suivante :
- Cire (PHYTOWAX Olive 18 L 57 de la société SOPHIM) 17 g
- Copolymère acétate de vinyle/stéarate d'allyle (65/35) (Mexomère PQ de CHIMEX) 7 g
- Quaternium-18 bentonite 5 g
- Propylène carbonate 1,6 g
- Oxyde de fer noir 5 g
- Isododécane qsp 100 g

Après application du mascara sur les cils, on obtient un très bon recourbement des cils.

### Exemple 4 :

On a préparé un mascara ayant la composition suivante :
- Dispersion de polymère de l'exemple 5 10 g
- Cire (PHYTOWAX Olive 18 L 57 de la société SOPHIM) 15 g
- Copolymère acétate de vinyle/stéarate d'allyle (65/35) (Mexomère PQ de CHIMEX) 2,2 g
- Polylaurate de vinyle (Mexomère PP de CHIMEX) 0,75 g
- Amidon de riz 0,84 g
- Bentonite 5,32 g
- Carbonate de propylène 1,74 g
- Pigments 4,6 g
- Conservateurs qs
- Isododécane qsp 100 g

### Exemple 5 de dispersion de polymère :

On a préparé une dispersion de copolymère non réticulé d'acrylate de méthyle et d'acide acrylique dans un rapport 95/5, dans de l'isododécane, selon la méthode de l'exemple 7 du document EP-A-749 747. On obtient ainsi une dispersion de particules de poly(acrylate de méthyle/acide acrylique) stabilisées en surface dans de l'isododécane par un copolymère dibloc séquencé polystyrène/copoly(éthylène-propylène) vendu sous le nom de KRATON G1701 (Shell), ayant un taux de matière sèche de 24,2% en poids et une taille moyenne des particules de 180 nm et une Tg de 20°C. Ce copolymère est filmifiable à température ambiante (25 °C).

## Revendications

1. Composition à application topique comprenant, dans un milieu physiologiquement acceptable, au moins une phase grasse liquide, contenant au moins une huile et au moins une cire, caractérisée par le fait que la cire a un point de fusion inférieur à 60 °C et une dureté, à 20 °C, supérieure ou égal à 9,5 MPa.

2. Composition selon la revendication 1, caractérisée par le fait que la cire a un point de fusion allant de 30 °C à 59 °C.

3. Composition selon la revendication 1 ou 2, caractérisée par le fait que la cire a un point de fusion allant de 35 °C à 59 °C, et mieux allant de 40 °C à 50 °C.

4. Composition selon l'une quelconque des revendications précédentes, caractérisée par le fait que la cire a une dureté allant de 9,5 MPa à 20 MPa.

5. Composition selon l'une quelconque des revendications précédentes, caractérisée par le fait que la cire a une dureté allant de 9,5 MPa à 15 MPa, et mieux allant de 10 à 12 MPa.

6. Composition selon l'une quelconque des revendications précédentes, caractérisée par le fait que la cire est choisie parmi les composés de formule (I) suivante : 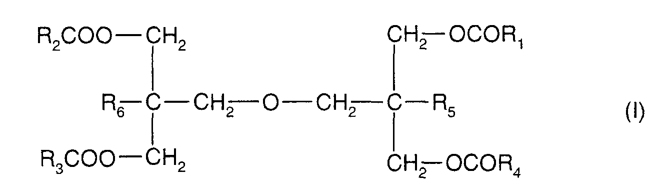 dans laquelle :
- R₁, R₂, R₃ et R₄ désignent, indépendamment l'un de l'autre, un radical alkyl linéaire ayant de 13 à 19 atomes de carbone, et de préférence de 15 à 17 atomes de carbone,
- R₅ et R₆ désignent, indépendamment l'un de l'autre, un radical alkyle choisi parmi les radicaux méthyle, éthyle et propyle.

7. Composition selon la revendication 6, caractérisée par le fait que R₁, R₂, R₃ et R₄ désignent un radical alkyle ayant 17 atomes de carbones.

8. Composition selon la revendication 6 ou 7, caractérisée par le fait que R₅ et R₆ désignent le radical méthyle.

9. Composition selon l'une quelconque des revendications précédentes, caractérisée par le fait que la cire est le tétrastéarate de di-(triméthylol-1,1,1 propane).

10. Composition selon l'une quelconque des revendications précédentes, caractérisée par le fait que la cire est obtenue par hydrogénation d'huile d'olive estérifiée par l'alcool stéarylique.

11. Composition selon l'une quelconque des revendications précédentes, caractérisée par le fait que la cire est présente en une teneur allant de 0,5 % à 60 % en poids, par rapport au poids total de la composition, de préférence de 1 % à 40 % en poids.

12. Composition selon l'une quelconque des revendications précédentes, caractérisée par le fait que la phase grasse liquide comprend au moins une huile volatile.

13. Composition selon la revendication 12, caractérisée par le fait que l'huile volatile est choisie dans le groupe formé par les huiles hydrocarbonées volatiles, les huiles de silicone volatile, les huiles fluorées volatiles.

14. Composition selon la revendication 12 ou 13, caractérisée par le fait que l'huile volatile est choisie parmi les isoparaffines comportant de 8 à 16 atomes de carbone.

15. Composition selon la revendication 12 ou 13, caractérisée par le fait que l'huile volatile est choisie dans le groupe formé par l'octaméthylcyclotétrasiloxane, le décaméthylcyclopentasiloxane, le dodécaméthylcyclohexasiloxane, l'octaméthyltrisiloxane, l'heptaméthylhexyltrisiloxane, l'heptaméthyloctyltrisiloxane, le décaméthyltétrasiloxane.

16. Composition selon l'une quelconque des revendications 12 à 15, caractérisée par le fait que l'huile volatile est présente en une teneur allant de 1 % à 99,5 % en poids, par rapport au poids total de la composition, et de préférence de 5 % à 98 % en poids.

17. Composition selon l'une quelconque des revendications précédentes, caractérisée par le fait qu'elle comprend en outre au moins une huile non volatile.

18. Composition selon l'une quelconque des revendications précédentes, caractérisée par le fait qu'elle comprend, en outre, au moins une cire additionnelle choisie dans le groupe formé par les cires ayant un point de fusion supérieur ou égal à 60 °C et les cires ayant un point de fusion inférieur à 60 °C et une dureté, à 20 °C, inférieure à 9,5 MPa.

19. Composition selon la revendication 18, caractérisée par le fait que la cire additionnelle a un point de fusion allant de 60 °C à 110 °C.

20. Composition selon la revendication 18 ou 19, caractérisée par le fait que la cire additionnelle est présente en une teneur allant de 0,1 % à 30 % en poids, par rapport au poids total de la composition.

21. Composition selon l'une quelconque des revendications précédentes, caractérisée par le fait qu'elle comprend en outre au moins un polymère filmogène dans la phase grasse liquide.

22. Composition selon la revendication 21, caractérisée par le fait que le polymère filmogène est choisi parmi les copolymères de formule (III): 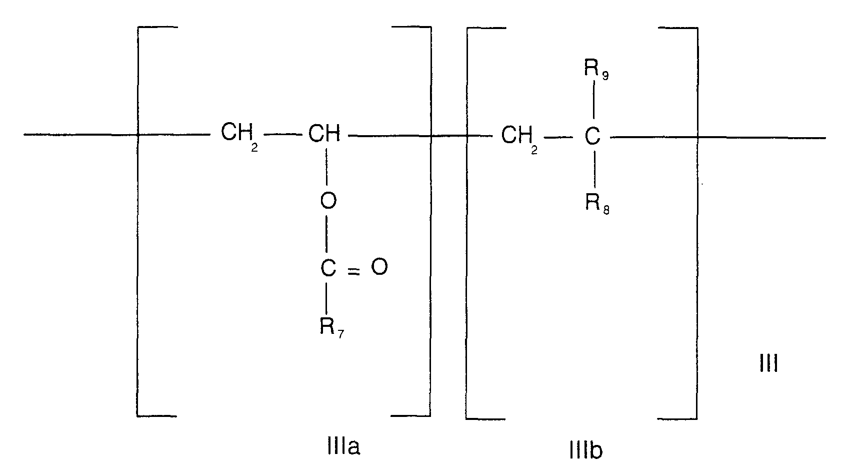 dans laquelle :
- R₇ représente une chaîne hydrocarbonée saturée, linéaire ou ramifiée, ayant de 1 à 19 atomes de carbone ;
- R₈ représente un radical pris dans le groupe constitué par :
a) -O-CO-R₁₀, R₁₀ ayant la même signification que R₇ mais est différent de R₇ dans un même copolymère,
b) -CH₂-R₁₁, R₁₁ représentant une chaîne hydrocarbonée saturée, linéaire ou ramifiée, ayant de 5 à 25 atomes de carbone,
c) -O-R₁₂, R₁₂ représentant une chaîne hydrocarbonée saturée, ayant de 2 à 18 atomes de carbone, et
d) -CH₂-O-CO-R₁₃, R₁₃ représentant une chaîne hydrocarbonée saturée, linéaire ou ramifiée, ayant de 1 à 19 atomes de carbone,
- R₉ représente un atome d'hydrogène quand R₈ représente les radicaux a), b) ou c) ou R₉ représente un radical méthyle quand R₈ représente le radical d), ledit copolymère devant être constitué d'au moins 15 % en poids d'au moins un monomère dérivé d'un motif (Illa) ou d'un motif (IIIb) dans lesquels les chaînes hydrocarbonées saturées, linéaires ou ramifiées, ont au moins 7 atomes de carbone.

23. Composition selon l'une des revendications 21 ou 22, caractérisée par le fait que le polymère filmogène est un homoplymère choisi dans le groupe formé par les homopolymères résultant de l'homopolymérisation d'esters vinyliques ayant de 9 à 22 atomes de carbone ou (méth)acrylates d'alkyle dont le radical alkyle a de 10 à 20 atomes de carbone.

24. Composition selon l'une quelconque des revendications 21 à 23, caractérisée par le fait que le polymère filmogène est choisi dans le groupe formé par les copolymères d'alcènes en C₂-C₂₀, les alkylcelluloses avec un radical alkyl linéaire ou ramifié, saturé ou non en C₁ à C₈, les copolymères de la vinylpyrrolidone.

25. Composition selon l'une quelconque des revendications 21 à 24, caractérisée par le fait que le polymère filmogène est choisi parmi les copolymères de la vinylpyrrolidone et d'alcène en C₂ à C₄₀.

26. Composition selon l'une quelconque des revendications 21 à 25, caractérisée par le fait que le polymère filmogène est liposoluble et est présent en une teneur allant de 0,1 % à 20 % en poids, par rapport au poids total de la composition, et de préférence de 0,5 % à 15 % en poids.

27. Composition selon l'une quelconque des revendications précédentes, caractérisée par le fait qu'elle comprend, en outre, au moins une dispersion de particules d'un système polymérique filmifiable ou non filmifiable comprenant un polymère, stabilisées en surface dans la phase grasse liquide.

28. Composition selon la revendication 27, caractérisée par le fait que le polymère en dispersion est choisi parmi les polymères radicalaires, les polycondensats, les polymères d'origine naturelle et leurs mélanges.

29. Composition selon la revendication 27 ou 28, caractérisée par le fait que le polymère en dispersion est choisi parmi les polyuréthannes, polyuréthannes-acryliques, polyurées, polyurée/polyuréthannes, polyester-polyuréthannes, polyéther-polyuréthannes, polyesters, polyesters amides, polyesters à chaîne grasse, alkydes ; polymères ou copolymères acryliques et/ou vinyliques ; polymères siliconés, polymères fluorés et leurs mélanges.

30. Composition selon l'une quelconque des revendications 27 à 29, caractérisée par le fait que le système polymérique comprend au moins un agent plastifiant.

31. Composition selon l'une quelconque des revendications 27 à 30, caractérisée par le fait que les particules du système polymérique sont stabilisées par un stabilisant choisi parmi les polymères séquencés, les polymères greffés, les polymères statistiques et leurs mélanges.

32. Composition selon la revendication 31, caractérisée par le fait que le stabilisant est choisi parmi les polymères siliconés greffés avec une chaîne hydrocarbonée ; les polymères hydrocarbonés greffés avec une chaîne siliconée ; les copolymères blocs greffés ou séquencés comprenant au moins un bloc de type polyorganosiloxane et au moins un bloc d'un polymère radicalaire ; les copolymères blocs greffés ou séquencés comprenant au moins un bloc de type polyorganosiloxane et au moins un bloc d'un polyéther ; les copolymères de (méth)acrylates d'alkyl en C₁-C₄ et de (méth)acrylates d'alkyl en C₈-C₃₀ ; les copolymères blocs greffés ou séquencés comprenant au moins un bloc résultant de la polymérisation de monomères éthyléniques comportant une ou plusieurs liaisons éthylénique éventuellement conjuguées et au moins un bloc d'un polymère styrénique ; les copolymères blocs greffés ou séquencés comprenant au moins un bloc résultant de la polymérisation de monomères éthyléniques et au moins un bloc d'un polymère acrylique ; les copolymères blocs greffés ou séquencés comprenant au moins un bloc résultant de la polymérisation de monomère éthylénique et au moins un bloc d'un polyéther, et leurs associations.

33. Composition selon la revendication 31 ou 32, caractérisée par le fait que le stabilisant est un copolymère bloc greffé ou séquencé, comprenant au moins un bloc résultant de la polymérisation de monomères éthyléniques comportant une ou plusieurs liaisons éthylénique éventuellement conjuguées et au moins un bloc d'un polymère styrénique.

34. Composition selon l'une quelconque des revendications 27 à 33, caractérisée par le fait que le polymère en dispersion dans la phase grasse liquide est présent en une teneur totale allant de 2 % à 60 % en poids, par rapport au poids total de la composition, de préférence de 4 % à 40 % en poids, et mieux de 5 % à 30 % en poids.

35. Composition selon l'une quelconque des revendications précédentes, caractérisée par le fait qu'elle comprend au moins une matière colorante choisie dans le groupe formé par les composés pulvérulents et les colorants liposolubles.

36. Composition selon la revendication 35, caractérisée par le fait que les composés pulvérulents sont choisis dans le groupe formé par les pigments, les nacres et les charges.

37. Composition cosmétique selon l'une quelconque des revendications précédentes, caractérisée par le fait qu'elle comprend au moins un agent épaississant de la phase grasse liquide.

38. Composition selon l'une quelconque des revendications précédentes, caractérisée par le fait qu'elle comprend au moins un additif choisi dans le groupe formé par les conservateurs, les parfums, les filtres solaires, les agents anti-radicaux libres, les agents hydratants, les vitamines, les protéines, les céramides, les plastifiants.

39. Composition selon l'une quelconque des revendications précédentes, caractérisée par le fait que la composition est anhydre.

40. Composition selon l'une quelconque des revendications 1 à 38, caractérisée par le fait qu'elle comprend moins de 10 % d'eau formant une phase aqueuse.

41. Composition selon la revendication 40, caractérisée par le fait que la phase aqueuse comprend un polymère filmogène additionnel sous forme de particules dispersées dans la phase aqueuse.

42. Composition selon l'une quelconque des revendications précédentes, caractérisée par le fait que la composition se présente sous la forme de composition de maquillage ou de traitement cosmétique des matières kératiniques.

43. Composition selon l'une quelconque des revendications précédentes, caractérisée par le fait qu'elle se présente sous la forme d'eye-liner, d'un produit de revêtement des fibres kératiniques, d'un fond de teint, d'un fard à paupières ou à joue, d'un rouge à lèvres, de vernis à ongles, d'un produit anti-cernes, ou encore d'un produit de maquillage du corps du type tatouage provisoire ou semi-permanent.

44. Composition selon l'une quelconque des revendications précédentes, caractérisée par le fait que la composition se présente sous la forme d'un mascara.

45. Procédé de maquillage des matières kératiniques, caractérisé par le fait que l'on applique sur les matières kératiniques une composition selon l'une quelconque des revendications 1 à 44.

46. Procédé de traitement non thérapeutique des matières kératiniques, caractérisé par le fait que l'on applique sur les matières kératiniques une composition selon l'une quelconque des revendications 1 à 44.

47. Utilisation d'une composition telle que définie selon l'une quelconque des revendications 1 à 44, pour recourber les fibres kératiniques, notamment les cils.
